# EUROPEAN PATENT SPECIFICATION

(11) **EP 4 037 570 B1**
(45) Date of publication and mention of the grant of the patent: **18.12.2024**
(21) Application number: 20873211.5
(22) Date of filing: 02.10.2020
(51) Int. Cl.: A61B 9/00, A61B 7/04, H01H 35/14

(54) **A MEDICAL TESTING APPARATUS**
MEDIZINISCHES TESTGERÄT
APPAREIL D'EXAMEN MÉDICAL

(30) Priority: 04.10.2019 FI 20195846
(43) Date of publication of application: 10.08.2022
(73) Proprietor: Danska Oy, 02420 Jorvas (FI)
(72) Inventor: DANSKA, Tomas, 02420 Jorvas (FI)
(74) Representative: Primrose Oy
(86) International application number: PCT/FI2020/050652
(87) International publication number: WO 2021/064296

(56) References cited:
- WO-A1-2019/060982
- WO-A2-2004/019770
- WO-A2-2012/022007
- CN-A- 105 769 251
- CN-A- 106 943 161
- CN-A- 108 514 433
- CN-A- 109 528 238
- CN-U- 204 500 779
- CN-U- 204 500 779
- CN-U- 204 863 281
- CN-U- 204 931 727
- US-A1- 2007 250 117
- US-A1- 2010 106 059
- US-A1- 2015 253 200
- US-A1- 2015 253 200
- US-B1- 6 406 436
- US-B1- 6 966 400

## Description

### FIELD OF THE INVENTION

The present invention relates to a medical testing apparatus comprising a body part and a handle part connected together as defined in independent claim 1.

### BACKGROUND OF THE INVENTION

A reflex hammer is a medical instrument which is used to test reflexes, and which is a basic instrument for a medical doctor. Medical doctors regularly need other basic instruments such as a light source for viewing the proximal mouth of a patient and a stethoscope for listening the lungs and heartbeat of a patient. The instruments need to be sterilized regularly to avoid contamination and transfer of contaminants from a patient to another. So, there are many instruments that are used regularly and many times a day and they have to be at hand and cleaned all the time.

Publication CN 109 528 238 discloses a multifunctional percussion hammer. Publication US 2015/253200 discloses an electronic thermometer. Publication CN 108 514 433 discloses an intelligent percussion hammer and publication CN 204 500 779 discloses a multifunctional percussion hammer.

Prior art documents CN 105 769 251 and CN 204 072 156 both disclose a medical testing apparatus comprising: a body part and a handle part connected together, the body part forming a reflex hammer having an impact surface, the medical testing apparatus further comprising:
a medical testing instrument which is electrically operable for medical testing;
a control unit in electrical communication with a power source and with the medical testing instrument; and the medical testing apparatus comprises:
   - a first axis defining a first operating direction of the medical testing apparatus which is the direction into which the reflex hammer is used; or
   - a first axis through the body part, the first axis extending between a first end having the impact surface of the reflex hammer and a second end which is opposite to the first end, the first axis defines a first operating direction of the medical testing apparatus;
wherein the medical testing instrument is a stethoscope having an operation surface for medical testing a human body, the stethoscope comprising a microphone for amplifying sound coming from said human body.

One of the problems associated with the prior art is that the number of instruments needed by a medical doctor is large and as they are used regularly during the day, a lot of time is required for cleaning the instruments.

### BRIEF DESCRIPTION OF THE INVENTION

An object of the present invention is to provide a medical testing apparatus which alleviates the problems of prior art and diminishes time spend on cleaning the instruments.

The invention relates to a medical testing apparatus as defined in independent claim 1. The preferred embodiments of the invention are disclosed in the dependent claims.

The invention is based on the idea of combining basic instruments that are needed by a medical doctor during a normal working day. A medical testing apparatus according to the invention comprises a body part and a handle part connected together. The body part forms a reflex hammer having an impact surface. The medical testing apparatus further comprises a medical testing instrument, a control unit and a motion sensor. The medical testing instrument is electrically operable for medical testing, which means that in order to function the medical testing instrument requires electricity, which is provided through a power source, for example battery, arranged within the medical testing apparatus. The battery can be a rechargeable battery which can be recharged through a charging point in the medical testing apparatus. The control unit is arranged in electrical communication with a power source and with the medical testing instrument and the motion sensor is provided in connection with the control unit. The motion senor forms a switch to the medical testing instrument such that the motion sensor is arranged to provide a first operating signal to the medical testing instrument to switch on the medical testing instrument for medical testing and a second operating signal to the medical testing instrument to switch off the medical testing instrument. In other words, the motion sensor switches on the medical testing instrument and switches off the medical testing instrument according to movements of the medical testing apparatus which the motion sensor detects. The motion sensor is preferably arranged to detect certain movements of the medical testing apparatus and to ignore other movements of the medical testing apparatus.

In an embodiment of the invention the medical testing apparatus comprises a first axis which is perpendicular to the impact surface of the reflex hammer and defines a first operating direction of the apparatus, and the motion sensor is arranged to provide the first operating signal to the medical testing instrument in response to a first movement of the apparatus other than the movement along the first axis of the apparatus, and the second operating signal in response to a second movement of the apparatus other than the movement along the first axis of the apparatus. Alternatively, the medical testing apparatus comprises a first axis through the body part which the first axis is extending between a first end having the impact surface of the reflex hammer and a second end which is opposite to the first end. The first axis defines a first operating direction of the apparatus, and the motion sensor is arranged to provide the first operating signal to the medical testing instrument in response to a first movement of the apparatus other than the movement along the first axis of the apparatus, and the second operating signal in response to a second movement of the apparatus other than the movement along the first axis of the apparatus.

In an embodiment of the invention the operating signals provided by the motion sensor to the medical testing instrument are provided in response to one or more movements of the medical testing apparatus in a direction other than along the first axis of the apparatus.

In an embodiment of the invention the motion sensor is arranged to recognize a first movement of the medical testing apparatus and to provide the first operating signal to the medical testing instrument in response to the first movement to switch on the medical testing instrument, and a second movement of the medical testing apparatus and to provide the second operating signal to the medical testing instrument in response to the second movement to switch off the medical testing instrument.

In an embodiment of the invention the one or more movements of the medical testing apparatus recognized by the motion sensor are linear movements. Alternatively, or in addition the one or more movements of the medical testing apparatus recognized by the motion sensor are rotational movements.

According to the present disclosure, but not falling within the scope of the claimed subject-matter, the medical testing instrument is a light unit arranged in the body part of the medical testing apparatus. Alternatively, the medical testing instrument is a light unit arranged in the handle part of the medical testing apparatus.

According to the present disclosure, the light unit is provided on the opposite end of the body part than the impact surface of the reflex hammer.

In an embodiment of the invention the control unit is arranged within the body part. Alternatively, the body part comprising a space for the control unit within the body part and a lid for closing the space and for forming a closed structure.

In an embodiment of the invention the body part comprises a space for the control unit within the body part and a lid for closing the space and for forming a closed structure. According to the present disclosure, the space further comprises a light unit provided within the space and said lid is transparent such that the light unit is operable through the transparent lid for transmitting light outside the apparatus. Alternatively, a light unit is provided within the space and said lid is a lens that amplifies the light from the light unit to outside of the apparatus.

In an embodiment of the invention the stethoscope also comprises a lid in a form of a membrane.

According to the claimed invention as defined in independent claim 1, the medical testing instrument is a stethoscope having an operational surface for medical testing a human body, the stethoscope comprising a microphone for amplifying sound coming from said human body and a transmitter for transferring the sound from the microphone to a separate receiver.

In an embodiment of the invention the stethoscope comprises a Bluetooth transmitter for transferring the sound through a Bluetooth connection from the microphone to a separate receiver comprising a Bluetooth receiver.

In an embodiment of the invention the stethoscope is arranged in the handle part of the medical testing apparatus such that the end of the handle part is arranged to form the operational surface for medical testing.

In an embodiment of the invention the body part of the medical testing apparatus is arranged to form a watertight housing for the control unit and the power source provided inside the body part. Alternatively, the medical testing instrument is provided to the body part of the medical testing apparatus in a watertight manner.

In an embodiment of the invention the handle part of the medical testing apparatus is arranged to form a watertight housing for the control unit and the power source provided inside the handle part. Alternatively, the medical testing instrument is provided to the handle part of the apparatus in a watertight manner.

In an embodiment of the invention the power source provided inside the medical testing instrument is a battery or a rechargeable or a disposable battery.

In an embodiment of the invention the motion sensor is an accelerometer.

The verb "amplify" means in the context of this application increasing intensity or range. The verb "amplify" is used in connection with a lens and a microphone as a common verb meaning mainly intensifying but also for example focusing, dispersing or converting.

An advantage of the invention is that the medical testing apparatus does not comprise an outsider switch for switching on and off the medical testing instrument such that the switch is arranged on a housing of the medical testing apparatus or on the outer surface of the medical testing apparatus. An advantage of the invention is that the medical testing apparatus forms a closed structure which is watertight and is therefore easy to clean. A further advantage is that time spent on cleaning the medical testing instruments is decreased because of combining the medical testing instruments into one medical testing apparatus and providing a closed structure for the medical testing apparatus which is watertight. A further advantage of the medical testing instrument is that it is a wireless device.

### BRIEF DESCRIPTION OF THE DRAWINGS

The invention is described in detail by means of specific embodiments with reference to the enclosed drawings, in which
Figure 1 shows an embodiment of a medical testing apparatus according to the invention;
Figure 2 shows the medical testing apparatus shown in figure 1 as an exploded view;
Figure 3 shows another embodiment of a medical testing apparatus according to the invention; and
Figure 4 shows the medical testing apparatus shown in figure 3 as an exploded view.

### DETAILED DESCRIPTION OF THE INVENTION

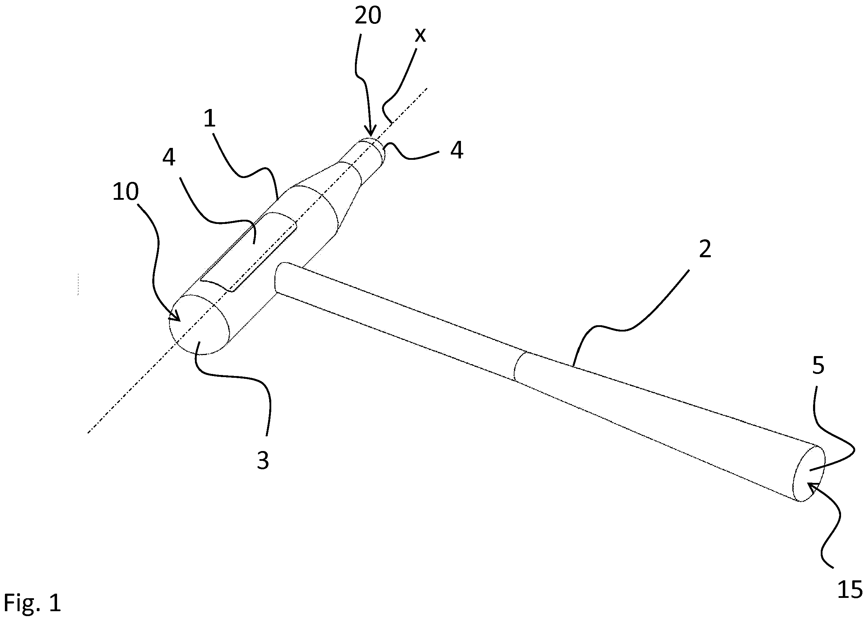

Figure 1 shows an embodiment of a medical testing apparatus according to the invention in which the medical testing apparatus comprises a body part 1 and a handle part 2 connected to the body part 1. The body part 1 comprises a reflex hammer 3 provided to a first end 10 of the body part 1. In this embodiment shown in figure 1 the impact surface of the reflex hammer is in a form of a hemisphere, but the impact surface may also be in another form for example flat or substantially flat. A first axis x extends through the body part 1 from the first end 10 to the second end 20. The second end 20 of the body part comprises in this embodiment of the invention a light unit 4 provided in connection with the body part 1. The light unit 4 is electrically operable and receives electricity from a power source provided within the body part 1. In other embodiments of the invention the power source may be provided within the handle part 2. The light unit 4 is arranged to provide light through the body part 1 such that the body part 1 comprises a transparent structure in the area of the light unit 4 or at least partially in the area of the light unit 4. The structure of the body part 1 is watertight also in the area of the light unit 4 such that the medical testing apparatus can be sanitized. In a preferable embodiment of the invention the light unit 4 comprises a lens forming part of the outer surface of the body part 1 and forming a watertight structure together with the body part 1. The lens is a transparent structure which gives refractive power to a light source provided in the light unit 4. The lens can be made for example of plastic, acrylic or polycarbonate. In an embodiment of the invention the light unit 4 is arranged to provide light in different colours so that for example patient's ability to react can be tested. The body part 1 and/or the handle part 2 is preferably made of aluminium or titanium. In another embodiment of the invention the body part 1 and/or the handle part 2 is coated with aluminium or titanium.

As can be seen from figure 1 the first axis x is arranged through the body part 1 such that it extends between the first end 10 having the impact surface of the reflex hammer 3 and a second end 20 which is opposite to the first end 10, and which comprises in this embodiment the light unit 4. The first axis x defines a first operating direction of the medical testing apparatus which the first operating direction is the direction into which the reflex hammer is used. The medical testing apparatus further comprises the motion sensor which is arranged to provide the first operating signal to the medical testing instrument 4, 5 in response to a first movement of the medical testing apparatus other than the movement along the first axis x of the medical testing apparatus, and the second operating signal in response to a second movement of the medical testing apparatus other than the movement along the first axis x of the medical testing apparatus. In other words, in order to give an operating signal to the medical testing instrument 4, 5, the medical testing instrument moves into a direction other than along the first axis of the medical testing apparatus and the motion sensor detects the movement and in response to the movement the motion sensor provides the operating signal to the medical testing instrument. The motion sensor may be arranged to give only two signals to the medical testing instrument: to switch on and off the medical testing instrument; or the motion sensor may be arranged give several signals to the medical testing instrument for different purposes. The operating signal from the motion sensor to the medical testing instrument may be a signal for switching on the medical testing instrument 4, 5 or for switching off the medical testing instrument, or the signal may be for dimming the light of the light unit 4 or changing colour of the light in the light unit 4.

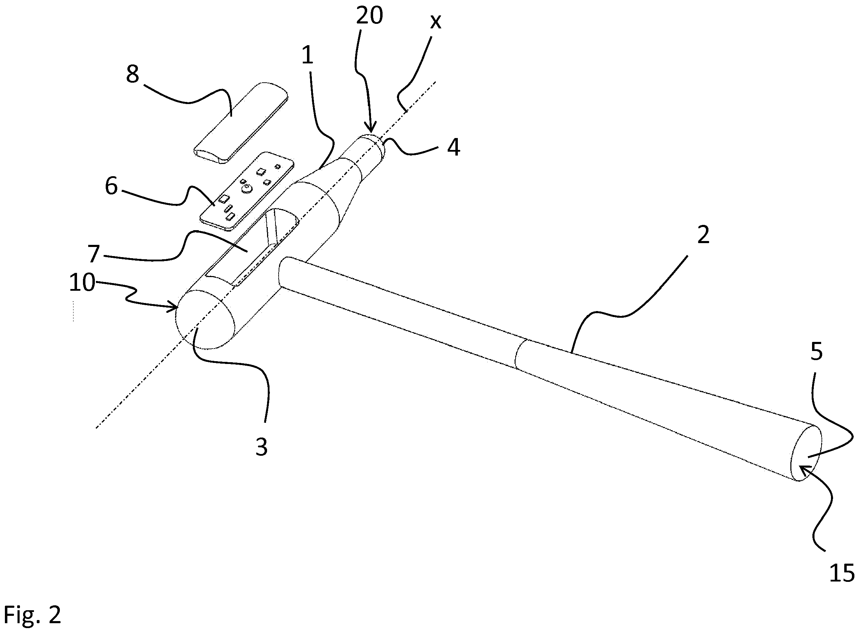

In the embodiment shown in figure 1 the handle part 2 comprises a stethoscope 5 having an operational surface 15 for medical testing of a human body. The stethoscope 5 comprising a microphone for amplifying sound coming from said human body and a transmitter for transferring the sound from the microphone to a separate receiver. The separate receiver can be for example an earpiece. The microphone and the transmitter can be arranged in connection with the control unit 6 (shown in figure 2). The control unit 6 preferably further comprises a printed circuit board and the motion sensor such that the motion sensor can be arranged in connection with the printed circuit board. The stethoscope 5 is arranged in the handle part 2 of the medical testing apparatus such that the end of the handle part 2 is arranged to form the operational surface 15 for medical testing. Transferring the sound from the microphone to the separate receiver is arranged to be wireless.

Switching on and off the stethoscope 5 is arranged through a movement detected by the motion sensor, which the movement is different than the movement for operating the light unit 4. In other words, movements of the medical testing apparatus for using the reflex hammer 3 along the first axis are different than the movements for operating the light unit 4 and different that the movement for operating the stethoscope 5 which the movements for operating the light unit 4 and the stethoscope 5 are also different. For example, in a normal use of a reflex hammer for reflex testing, the motion sensor does not control the light unit or the stethoscope, but when the medical testing apparatus is moved for example at a 90° angle from a normal operating direction for the reflex hammer, the light unit or the stethoscope operates. In other words, the motion sensor is arranged to form a switch for the light unit 4 and a switch for the stethoscope.

Figure 1 shows an alternative placement for the light unit 4 such that the light unit 4 is provided in the body part 1 between the first end 10 and the second end 20 of the body part 1. The light unit 4 provides therefore light direction which is transverse relative to the impact surface and preferably perpendicular to the impact surface. In other words, figure 1 shows two placements for the light unit 4.

Figure 2 shows the medical testing apparatus shown in figure 1 as an exploded view in which the control unit 6 and a lid 8 covering the control unit 6 are separated from the body part 1. The body part 1 comprises a space 7 for the control unit 6 within the body part 1 and a lid 8 for closing the space 7 and for forming a closed structure. The lid 8 is arranged in connection with the body part 1 such that a watertight structure is formed. In an embodiment of the invention the space 7 may further comprise a light unit 4 provided within the space 7 and said lid 8 is transparent such that the light unit is operable through the transparent lid 8 for transmitting light outside the medical testing apparatus. Alternatively, the light unit 4 is provided within the space 7 and said lid 8 is a lens that amplifies the light from the light unit 4 to outside of the medical testing apparatus. In another example of the invention the light unit 4 is arranged within the space 7 and the second end of the body part 1 is arranged to be transparent or comprises a lens such that the space 7 extends at least partially to the second end of the body part 1 for transmitting light from the light unit through the transparent second end 20 of the body part 1 or through the second end 20 comprising the lens to outside of the medical testing apparatus.

The control unit 6 preferably comprises the printed circuit board together with the motion sensor and preferably with the microphone and the transmitter. The space 7 may further comprise the power source such as battery.

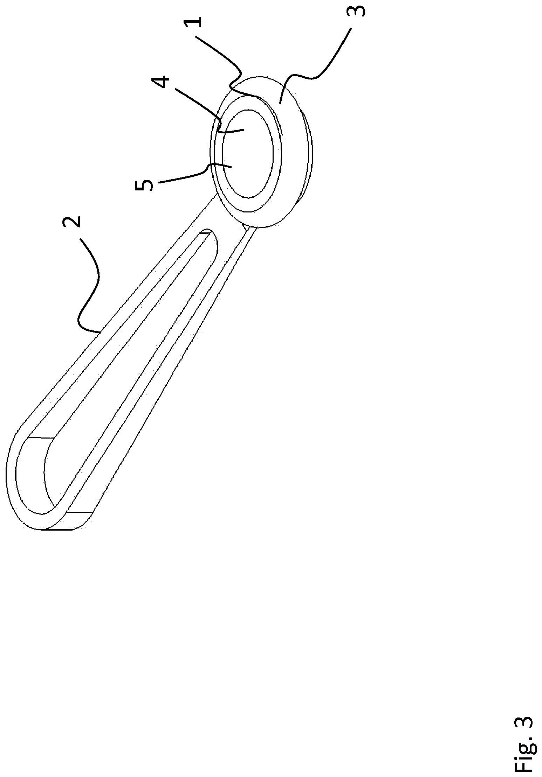

Figure 3 shows another embodiment of the medical testing apparatus according to the invention in which the body part 1 is extending from the handle part 2 and forming the reflex hammer 3 on the outer surface of the body part 2. The reflex hammer 3 is provided in a form of rubber ring around the body part 2. The body part 2 further comprises the light unit 4 and the stethoscope 5 in a more compact form than presented in figures 1 and 2. In the embodiment shown in figure 3 the light unit 4 and the stethoscope 5 are provided superimposed, i.e. the stethoscope 5 is provided on top of the light unit 4 whereby the stethoscope 5 having a membrane that is light transmitting or transparent. However, the light unit 4 and the stethoscope 5 may be provided superimposed such that the light unit 4 is provided on the opposite side of the body part 2 than the stethoscope 5.

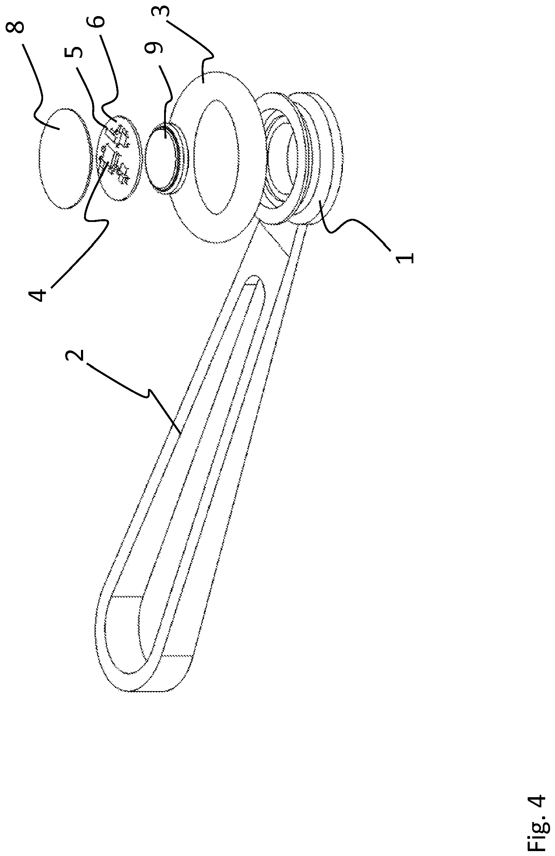

Figure 4 shows the medical testing apparatus shown in figure 3 as an exploded view in which the body part 2 is in a dissembled state. The rubber ring forming the reflex hammer 3 is provided as an O-ring and surrounds the periphery of the body part 2 from the outer surface of the body part 2 in an assembled state. The power source 9 and the control unit 6 together with the light source 4 are provided within the body part 2. The lid 8 closing the power source 9, the control unit 6, the light source 4 and/or the microphone within the body part 2 can be formed as a lens amplifying light coming from the light source or a membrane forming the operational surface for the stethoscope. The light source and the microphone can be provided in connection with the control unit 6 such that they are on same side of the control unit or opposite sides of the control unit such that the light unit 4 and the stethoscope 5 are superimposed either on the same end of the body part 2 or opposite ends of the body part 2. In the embodiment of the medical testing apparatus shown in figures 3 and 4 the first operating direction of the medical testing apparatus is perpendicular to the direction of light coming from the light source 4.

## Claims

1. A medical testing apparatus comprising a body part (1) and a handle part (2) connected together, the body part (1) forming a reflex hammer (3) having an impact surface, the medical testing apparatus further comprising:
a medical testing instrument (4, 5) which is electrically operable for medical testing;
a control unit (6) in electrical communication with a power source and with the medical testing instrument (4, 5); and
a motion sensor provided in connection with said control unit (6), the motion sensor forming a switch to the medical testing instrument (4, 5) such that the motion sensor is arranged to provide a first operating signal to the medical testing instrument (4, 5) to switch on the medical testing instrument (4, 5) for medical testing and a second operating signal to the medical testing instrument (4, 5) to switch off the medical testing instrument (4, 5),
the medical testing apparatus comprises:
- a first axis (x) defining a first operating direction of the medical testing apparatus which is the direction into which the reflex hammer is used; or
- a first axis (x) through the body part (1), the first axis (x) extending between a first end (10) having the impact surface of the reflex hammer (3) and a second end (20) which is opposite to the first end (10), the first axis (x) defines a first operating direction of the medical testing apparatus;
the medical testing apparatus comprising the motion sensor which is arranged to provide the first operating signal to the medical testing instrument (4, 5) in response to a first movement of the medical testing apparatus other than the movement along the first axis (x) of the medical testing apparatus, and the second operating signal in response to a second movement of the medical testing apparatus other than the movement along the first axis (x) of the medical testing apparatus,
the medical testing instrument (4, 5) is a stethoscope having an operational surface for medically testing a human body, the stethoscope comprising a microphone for amplifying sound coming from said human body and a transmitter for transferring the sound from the microphone to a separate receiver.

2. A medical testing apparatus according to claim 1, wherein the operating signals provided by the motion sensor to the medical testing instrument (4, 5) are provided in response to one or more movements of the medical testing apparatus in a direction other than along the first axis (x) of the medical testing apparatus.

3. A medical testing apparatus according to any previous claim, wherein the motion sensor is arranged to recognize
a first movement of the medical testing apparatus and to provide the first operating signal to the medical testing instrument (4, 5) in response to the first movement to switch on the medical testing instrument (4, 5), and
a second movement of the medical testing apparatus and to provide the second operating signal to the medical testing instrument (4, 5) in response to the second movement to switch off the medical testing instrument (4, 5).

4. A medical testing apparatus according to claim 2, wherein the one or more movements of the medical testing apparatus recognized by the motion sensor are linear movements; or
the one or more movements of the medical testing apparatus recognized by the motion sensor are rotational movements.

5. A medical testing apparatus according to any previous claim, wherein the control unit (6) is arranged within the body part (1); or
the body part (1) comprising a space (7) for the control unit (6) within the body part (1) and a lid (8) for closing the space (7) and for forming a closed structure.

6. A medical testing apparatus according to claim 1, wherein the stethoscope is arranged in the handle part (2) of the medical testing apparatus such that the end of the handle part (2) is arranged to form the operational surface for medical testing.

7. A medical testing apparatus according to any previous claim, wherein the body part (1) of the medical testing apparatus is arranged to form a watertight housing for the control unit (6) and the power source provided inside the body part (1); or
the medical testing instrument (4, 5) is provided to the body part (1) of the medical testing apparatus in a watertight manner.

8. A medical testing apparatus according to any of claims 1-6, wherein the handle part (2) of the medical testing apparatus is arranged to form a watertight housing for the control unit (6) and the power source provided inside the handle part (2); or
the medical testing instrument (4, 5) is provided to the handle part (2) of the medical testing apparatus in a watertight manner.

9. A medical testing apparatus according to any previous claim, wherein the motion sensor is an accelerometer.

## Patentansprüche

1. Medizinische Testeinrichtung, die einen Körperteil (1) und einen Griffteil (2) umfasst, die miteinander verbunden sind, wobei der Körperteil (1) einen Reflexhammer (3) bildet, der eine Schlagfläche aufweist, wobei die medizinische Testeinrichtung weiter Folgendes umfasst:
ein medizinisches Testinstrument (4, 5), das für medizinische Tests elektrisch betreibbar ist;
eine Steuereinheit (6) in elektrischer Kommunikation mit einer Leistungsquelle und mit dem medizinischen Testinstrument (4, 5); und
einen Bewegungssensor, der in Verbindung mit der Steuereinheit (6) bereitgestellt ist, wobei der Bewegungssensor einen Schalter für das medizinische Testinstrument (4, 5) bildet, so dass der Bewegungssensor dazu eingerichtet ist, ein erstes Betriebssignal an das medizinische Testinstrument (4, 5) bereitzustellen, um das medizinische Testinstrument (4, 5) für medizinische Tests einzuschalten, und ein zweites Betriebssignal an das medizinische Testinstrument (4, 5) bereitzustellen, um das medizinische Testinstrument (4, 5) auszuschalten,
wobei die medizinische Testeinrichtung Folgendes umfasst:
- eine erste Achse (x), die eine erste Betriebsrichtung der medizinischen Testeinrichtung definiert, die die Richtung ist, in der der Reflexhammer verwendet wird; oder
- eine erste Achse (x) durch den Körperteil (1), wobei die erste Achse (x) zwischen einem ersten Ende (10), das die Schlagfläche des Reflexhammers (3) aufweist, und einem zweiten Ende (20) verläuft, das dem ersten Ende (10) gegenüberliegt, wobei die erste Achse (x) eine erste Betriebsrichtung der medizinischen Testeinrichtung definiert;
wobei die medizinische Testeinrichtung den Bewegungssensor umfasst, der so eingerichtet ist, dass er als Reaktion auf eine erste Bewegung der medizinischen Testeinrichtung, die nicht die Bewegung entlang der ersten Achse (x) der medizinischen Testeinrichtung ist, das erste Betriebssignal, und als Reaktion auf eine zweite Bewegung der medizinischen Testeinrichtung, die nicht die Bewegung entlang der ersten Achse (x) der medizinischen Testeinrichtung ist, das zweite Betriebssignal an das medizinische Testinstrument (4, 5) bereitstellt,
das medizinische Testinstrument (4, 5) ein Stethoskop mit einer Bedienoberfläche zum medizinischen Testen eines menschlichen Körpers ist, wobei das Stethoskop ein Mikrofon zum Verstärken des von dem menschlichen Körper ausgehenden Schalls und einen Sender zum Übertragen des Schalls von dem Mikrofon an einen separaten Empfänger umfasst.

2. Medizinische Testeinrichtung nach Anspruch 1,
wobei die von dem Bewegungssensor an das medizinische Testinstrument (4, 5) bereitgestellten Betriebssignale als Reaktion auf eine oder mehrere Bewegungen der medizinischen Testeinrichtung in eine andere Richtung als entlang der ersten Achse (x) der medizinischen Testeinrichtung bereitgestellt werden.

3. Medizinische Testeinrichtung nach einem vorstehenden Anspruch,
wobei der Bewegungssensor dazu eingerichtet ist, Folgendes zu erkennen
eine erste Bewegung der medizinischen Testeinrichtung, und als Reaktion auf die erste Bewegung das erste Betriebssignal an das medizinische Testinstrument (4, 5) bereitzustellen, um das medizinische Testinstrument (4, 5) einzuschalten, und
eine zweite Bewegung der medizinischen Testeinrichtung, und als Reaktion auf die zweite Bewegung das zweite Betriebssignal an das medizinische Testinstrument (4, 5) bereitzustellen, um das medizinische Testinstrument (4, 5) auszuschalten.

4. Medizinische Testeinrichtung nach Anspruch 2,
wobei die eine oder mehreren Bewegungen der medizinischen Testeinrichtung, die von dem Bewegungssensor erkannt werden, lineare Bewegungen sind; oder
die eine oder mehreren Bewegungen der medizinischen Testeinrichtung, die von dem Bewegungssensor erkannt werden, Drehbewegungen sind.

5. Medizinische Testeinrichtung nach einem vorstehenden Anspruch,
wobei die Steuereinheit (6) innerhalb des Körperteils (1) eingerichtet ist; oder
wobei der Körperteil (1) einen Raum (7) für die Steuereinheit (6) innerhalb des Körperteils (1) und einen Deckel (8) zum Verschließen des Raums (7) und zum Bilden einer geschlossenen Struktur umfasst.

6. Medizinische Testeinrichtung nach Anspruch 1,
wobei das Stethoskop im Griffteil (2) der medizinischen Testeinrichtung eingerichtet ist, so dass das Ende des Griffteils (2) so angeordnet ist, dass es die Bedienfläche für medizinische Tests bildet.

7. Medizinische Testeinrichtung nach einem vorstehenden Anspruch,
wobei der Körperteil (1) der medizinischen Testeinrichtung eingerichtet ist, ein wasserdichtes Gehäuse für die Steuereinheit (6) und die innerhalb des Körperteils (1) bereitgestellte Leistungsquelle zu bilden; oder
das medizinische Testinstrument (4, 5) wasserdicht am Körperteil (1) der medizinischen Testeinrichtung bereitgestellt ist.

8. Medizinische Testeinrichtung nach einem der Ansprüche 1-6,
wobei der Griffteil (2) der medizinischen Testeinrichtung eingerichtet ist, ein wasserdichtes Gehäuse für die Steuereinheit (6) und die innerhalb des Griffteils (2) bereitgestellte Leistungsquelle zu bilden; oder
das medizinische Testinstrument (4, 5) wasserdicht am Griffteil (2) der medizinischen Testeinrichtung bereitgestellt ist.

9. Medizinische Testeinrichtung nach einem vorstehenden Anspruch,
wobei der Bewegungssensor ein Beschleunigungsmesser ist.

## Revendications

1. Appareil d'examen médical comprenant une partie corps (1) et une partie manche (2) reliées ensemble, la partie corps (1) formant un marteau à réflexes (3) présentant une surface de choc, l'appareil d'examen médical comprenant en outre :
un instrument d'examen médical (4, 5) qui peut être actionné électriquement pour un examen médical ;
une unité de commande (6) en communication électrique avec une source d'énergie et avec l'instrument d'examen médical (4, 5) ; et
un capteur de mouvement disposé en liaison avec ladite unité de commande (6), le capteur de mouvement formant un commutateur pour l'instrument d'examen médical (4, 5) de telle sorte que le capteur de mouvement soit agencé pour fournir un premier signal d'actionnement à l'instrument d'examen médical (4, 5) pour allumer l'instrument d'examen médical (4, 5) pour un examen médical et un second signal d'actionnement à l'instrument d'examen médical (4, 5) pour éteindre l'instrument d'examen médical (4, 5),
l'appareil d'examen médical comprend :
- un premier axe (x) définissant une première direction de fonctionnement de l'appareil d'examen médical qui est la direction dans laquelle le marteau à réflexes est utilisé ; ou
- un premier axe (x) traversant la partie corps (1), le premier axe (x) s'étendant entre une première extrémité (10) présentant la surface de choc du marteau à réflexes (3) et une seconde extrémité (20) qui est opposée à la première extrémité (10), le premier axe (x) définit une première direction de fonctionnement de l'appareil d'examen médical ;
l'appareil d'examen médical comprenant le capteur de mouvement qui est agencé pour fournir le premier signal d'actionnement à l'instrument d'examen médical (4, 5) en réponse à un premier mouvement de l'appareil d'examen médical autre que le mouvement le long du premier axe (x) de l'appareil d'examen médical, et le second signal d'actionnement en réponse à un second mouvement de l'appareil d'examen médical autre que le mouvement le long du premier axe (x) de l'appareil d'examen médical,
l'instrument d'examen médical (4, 5) est un stéthoscope présentant une surface fonctionnelle pour examiner médicalement un corps humain, le stéthoscope comprenant un microphone pour amplifier le son provenant dudit corps humain et un émetteur pour transférer le son du microphone à un récepteur séparé.

2. Appareil d'examen médical selon la revendication 1,
dans lequel les signaux d'actionnement fournis par le capteur de mouvement à l'instrument d'examen médical (4, 5) sont fournis en réponse à un ou plusieurs mouvements de l'appareil d'examen médical dans une direction autre que le long du premier axe (x) de l'appareil d'examen médical.

3. Appareil d'examen médical selon une quelconque revendication précédente,
dans lequel le capteur de mouvement est agencé pour reconnaître
un premier mouvement de l'appareil d'examen médical et pour fournir le premier signal d'actionnement à l'instrument d'examen médical (4, 5) en réponse au premier mouvement pour allumer l'instrument d'examen médical (4, 5), et
un second mouvement de l'appareil d'examen médical et pour fournir le second signal d'actionnement à l'instrument d'examen médical (4, 5) en réponse au second mouvement pour éteindre l'instrument d'examen médical (4, 5).

4. Appareil d'examen médical selon la revendication 2,
dans lequel les un ou plusieurs mouvements de l'appareil d'examen médical reconnus par le capteur de mouvement sont des mouvements linéaires ; ou
les un ou plusieurs mouvements de l'appareil d'examen médical reconnus par le capteur de mouvement sont des mouvements de rotation.

5. Appareil d'examen médical selon une quelconque revendication précédente,
dans lequel l'unité de commande (6) est agencée à l'intérieur de la partie corps (1) ; ou
la partie corps (1) comprenant un espace (7) pour l'unité de commande (6) à l'intérieur de la partie corps (1) et un couvercle (8) pour fermer l'espace (7) et pour former une structure fermée.

6. Appareil d'examen médical selon la revendication 1,
dans lequel le stéthoscope est agencé dans la partie manche (2) de l'appareil d'examen médical de telle sorte que l'extrémité de la partie manche (2) soit agencée pour former la surface fonctionnelle pour l'examen médical.

7. Appareil d'examen médical selon une quelconque revendication précédente,
dans lequel la partie corps (1) de l'appareil d'examen médical est agencée pour former un boîtier étanche pour l'unité de commande (6) et la source d'énergie disposée à l'intérieur de la partie corps (1) ; ou
l'instrument d'examen médical (4, 5) est disposé sur la partie corps (1) de l'appareil d'examen médical de manière étanche.

8. Appareil d'examen médical selon l'une quelconque des revendications 1-6,
dans lequel la partie manche (2) de l'appareil d'examen médical est agencée pour former un boîtier étanche pour l'unité de commande (6) et la source d'énergie disposée à l'intérieur de la partie manche (2) ; ou
l'instrument d'examen médical (4, 5) est disposé sur la partie manche (2) de l'appareil d'examen médical de manière étanche.

9. Appareil d'examen médical selon une quelconque revendication précédente, dans lequel le capteur de mouvement est un accéléromètre.
